# EUROPÄISCHE PATENTSCHRIFT

(11) **EP 0 998 216 B1**
(45) Veröffentlichungstag und Bekanntmachung des Hinweises auf die Patenterteilung: **09.06.2004**
(21) Anmeldenummer: 98945089.5
(22) Anmeldetag: 23.07.1998
(51) Int. Cl.: A61B 5/05

(54) **ANORDNUNG ZUM NACHWEIS, ZUR DIFFERENTIALDIAGNOSTISCHEN CHARAKTERISIERUNG UND ZUR THERAPIE VON TUMOREN**
DEVICE FOR DETECTING, CHARACTERIZING BY DIFFERENTIAL DIAGNOSIS, AND TREATING TUMOURS
AGENCEMENT POUR LA DETECTION, LA CARACTERISATION PAR DIAGNOSTIC DIFFERENTIEL ET LE TRAITEMENT DE TUMEURS

(30) Priorität: 25.07.1997 DE 19732067; 25.07.1997 DE 19732068
(43) Veröffentlichungstag der Anmeldung: 10.05.2000
(73) Patentinhaber: Kaiser, Werner A., 53757 St. Augustin (DE)
(72) Erfinder: Kaiser, Werner A., 53757 St. Augustin (DE)
(74) Vertreter: Bock, Gerhard
(86) Internationale Anmeldenummer: PCT/EP1998/004615
(87) Internationale Veröffentlichungsnummer: WO 1999/004689

(56) Entgegenhaltungen:
- WO-A-97/36192
- WO-A-98/23204
- DE-A- 4 300 018
- DE-A- 4 344 986
- GB-A- 2 019 579

## Beschreibung

Die Erfindung betrifft eine. Anordnung zum Nachweis, zur differentialdiagnostischen. Charakterisierung. und zur Therapie von Tumoren gemäß der Gattung der Patentansprüche. Eine derartige Anordnung ist insbesondere zur Behandlung von Mammakarzinomen geeignet.

Aus der DE 42 43 628 A1 ist eine Vorrichtung zur nichtinvasiven Bestimmung der räumlichen Verteilung der elektrischen Impedanz im Inneren eines Lebewesens bekannt. Ebenso ist ein Verfahren der elektrischen Impedanzabbildung an tierischen oder menschlichen Lebewesen unter Verwendung von ionisierten Metallchelaten bereits aus den US-PS 5,651,955 und US-PS 5,733,525 bekannt, bei dem geeignete Kontrastmittel benutzt und wenigstens ein Teil eines Lebewesens impedanzmäßig abgebildet wird Eine sich unmittelbar anschließende therapeutische Behandlung ist dort in keinem Fall vorgesehen.
Ferner ist die Verwendung von Nadeln zur transurethralen thermischen Ablation von Hyperplasien der Prostata bekannt, siehe The Journal of Urology, Vol. 156 (August 1996) pp 413 - 419. Mit der Erzeugung von Widerstandsbildem eines Organismus bzw. eines Organs ist diese Verwendung nicht gekoppelt.

Durch die Erfindung soll nicht nur eine gewebeschonende, tumorselektive Abbildung, sondern auch eine Verkürzung der Behandlungsdauer und/oder eine Ausdehnung der Behandlung auf größere Gewebebereiche erreicht werden. Das triff sowohl für die Diagnose als auch für die Therapie zu, die sich unmittelbar an die Diagnose anschließt, die also vorteilhafterweise am selben Widerstandsbild vorgenommen wird wie die Diagnose.

Gemäß der Erfindung wird diese Aufgabe durch die Merkmale der Patentansprüche gelöst. Die Erzeugung und Auswertung der Impedanzbilder eines Organismus oder Organismusteils kann entweder vor oder nach der Zuführung der Elektrolytlösung oder nur nach der Zuführung der Elektrolytlösung, aber vor dem therapeutischen Behandlungsprozeß erfolgen. Im letzteren Fall muss ein tumortypischer Schwellwert vorher definiert worden sein. Insbesondere wirkt sich die Anreicherung der Elektrolytlösung, die vorzugsweise eine physiologische Kochsalzlösung ist, günstig auf die Diagnose und die unmittelbar angeschlossene Therapie von Tumoren aus. Es wird dabei das Widerstandsbild eines Gewebes analog oder digital sichtbar gemacht und durch induktive Erhitzung gezielt inaktiviert. Dabei kann die Behandlung durch eine roboterartige Steuerung der Nadeln erfolgen.

Die Erfindung wird nachstehend an Hand der schematischen Zeichnung dreier Ausführungsbeispiele näher erläutert. Es zeigen:
- Fig. 1: eine gleichstrombetriebene erfindungsgemäße Anordnung,
- Fig. 2: einen Querschnitt einer wechselstrombetriebenen erfindungsgemäßen Anordnung und
- Fig. 3: eine Frontansicht eines Katheters.

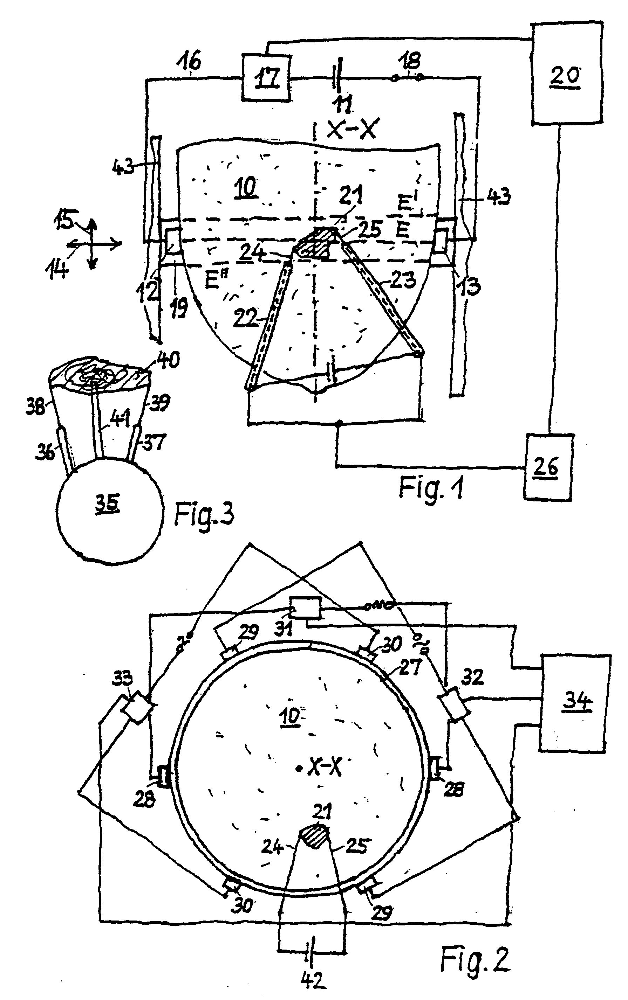

In Fig. 1 ist mit 10 eine weibliche Brust bezeichnet, an die zwei an eine Gleichstromquelle 11 angeschlossene, vorzugsweise diametral angeordnete Elektroden 12, 13 angelegt sind. Die Elektroden sind mit oder auf ihrem Träger 19 in durch Doppelpfeile 14, 15 gekennzeichneten Richtungen relativ zur Brust 10 linear verschiebbar und um eine in der Zeichenebene liegende Achse X-X drehbar. Der Träger 19 ist seinerseits in Führungen 43 parallel zur Achse X-X verstellbar. Im Gleichstromkreis 16 sind ein Meßinstrument 17 und ein Ein- und Aus-Schalter 18 angeordnet. Das Meßinstrument 17 ist mit einer Auswerteeinrichtung 20 verbunden, in der die vom Meßinstrument 17 kommenden Meßdaten ausgewertet und Widerstandsbilder erzeugt werden.
Durch eine gegenläufige Verschiebung der Elektroden 12, 13 relativ zum bzw. mit dem Träger 19 in Richtung des Doppelpfeiles 14 sind die Elektroden 12, 13 auf die Oberfläche der Brust 10 aufsetzbar. Die Drehung des Trägers 19 um die Achse X-X ermöglicht, in beliebigen Schritten die Elektroden 12, 13 in einer zur Zeichenebene rechtwinkligen Ebene E zu drehen und in diesen Schritten Meßdaten über den Widerstand in dieser Scheibe der Brust 10 zu gewinnen. Aus diesen Meßdaten wird in der Auswerteeinrichtung 20 durch Fouriertransformation und mathematische Faltung (E. Kerstel, Bildgebende Systeme für die medizinische Diagnostik, 2. Auflage 1988, Siemens AG, Berlin/München, Seiten 95 - 137) das Widerstandsbild der Ebene E erzeugt und sichtbar gemacht, das die zweidimensionale Widerstandsverteilung in der Scheibe erkennen läßt Stark leitende Objektbereiche entsprechen geringen Widerstandswerten, schwach leitende Objektbereiche entsprechen hohen Widerstandswerten. In gleicher Weise können auch Spannungswerte abgeleitet werden.
Durch Verschieben der Elektroden 12, 13 mit oder auf dem Träger 19 parallel zur Achse X-X werden Widerstandsbilder in weiteren Ebenen (Scheiben) E', E" in Analogie zur Bildherstellung bei der Computertomographie durch die Absorption von Röntgenstrahlen hergestellt und im Computer der Auswerteeinrichtung 20 berechnet Beim Vorhandensein mindestens eines Tumors 21 weichen die Widerstandsbilder voneinander oder von einem hier nicht dargestellten Testbild ab und lassen die Lage und Größe des Tumors deutlich erkennen. Um auch kleine Tumore erfassen zu können, müssen die Ebenen E, E', E" usw. genügend nahe beieinander liegen.
Zur Bekämpfung des Tumors 21 werden in Schutzhüllen 22, 23 befindliche Nadeln 24, 25 in den Organismusteil 10 eingeführt, die mit Hilfe von Radiofrequenzen das zwischen ihnen befindliche Gewebe über eine vorgewählte Dauer oder in Abhängigkeit von einem aktuell erzeugten und an einem Monitor der Auswerteeinrichtung 20 beobachtbaren Bild des Tumors 21 erhitzen. Dieses aktuelle Bild ermöglicht es, den Einstechvorgang der Nadeln 24, 25 und die Inaktivierung des Tumors 21 während seiner Behandlung zu verfolgen.
Die an eine entsprechende Energiequelle (bspw. Strom oder Radiofrequenz) angeschlossenen Nadeln 24, 25 können entweder von Hand oder durch eine automatische, roboterartige Steuerung 26 auf Grund der von der Auswerteeinrichtung 20 gelieferten Widerstandsbilder geführt werden. Damit sind auch sehr kleine Tumore präzise behandelbar.
Wird dem Organismus oder Organismusteil 10 eine Elektrolytlösung, bspw. physiologische Kochsalzlösung, intraarteriell, intravenös, z. B. über den Arm, oder direkt injiziert, so wird diese Lösung auf Grund der Tumorangiogenese im Tumor 21 initial verstärkt angereichert. Mit Hilfe der vorstehend beschriebenen Anordnung ist die Änderung des elektrischen Widerstands, den das Organismusteil 10 einem elektrischen Stromfluß entgegensetzt, parallel zur Anreicherung der Elektrolytlösung an Hand der Widerstandsmessung, der vom Meßinstrument 17 gelieferten Daten, am Widerstandsbild der Answerteeinrichtung 20 festzustellen. Durch die verstärkte Anreicherung der Elektrolytlösung in den ersten Minuten nach der Injektion kann eine relativ eindeutige Beurteilung eines Herdbefundes als maligne oder benigne vorgenommen werden. Dies stellt eine Analogie zu Daten aus der Bildgebung dar (siehe W. A. Kaiser, E. Zeitler. MR-imaging of the Breast: Fast Imaging Sequences with and without Gd-DTPA. Radiology 170 (1989), pp. 681-686).
Ggf. kann eine Subtraktion der Widerstände oder Widerstandsbilder mit und ohne Elektrolytinjektion in der Auswerteeinrichtung 20 den Effekt der Tumordarstellung und -lokalisierung verstärken. Bei stark vaskularisierten Tumoren kann ein reines Postkontrastbild ohne vorherige Nativmessung bereits einen Widerstand unter einem tumortypischen Schwellwert ergeben. Bei der Bekämpfung des lokalisierten Tumors 21 werden die beiden (oder mehrere) in das Gewebe von außen eingeführten Nadeln 24, 25 mit dem Tumor kontaktiert oder in die unmittelbare Nachbarschaft des Tumors gebracht. Durch die Strom- und/oder Radiofrequenzzufuhr von einer nicht dargestellten Quelle wird also der Tumor 21 inaktiviert, durch die Alteration von Membranpotentialen oder durch Hyperthermieeinwirkung. Diese Inaktivierung wird durch weitere Zugabe, z. B. direkte Injektion über eine Kanüle, von Elektrolytlösung vezstärkt und/oder beschleunigt Dabei dient die Elektrolytinjektion insbesondere dazu, den thermischen Effekt durch Strom- und/oder Radiofrequenzzufuhr einheitlicher und planbarer zu gestalten.

In Fig. 2 ist ein Organismusteil 10 zur Beibehaltung einer während der Messung und Behandlung konstanten Form von einer starren, elektrisch isolierenden Hülle 27 umgeben, die korbartig gestaltet sein kann, um durch ihre Wandöffnungen Nadeln 24, 25 zur Behandlung hindurchführen zu können, die an einer Gleichspannungsquelle 42 liegen. Um die Hülle 27 herum sind in einer Querschnittsebene E drei (oder vorzugsweise mehr) Elektrodenpaare 28, 29, 30, parallel zu einer Achse X-X verstellbar, in regelmäßigen Abständen angeordnet, die mit Wechselstrom betrieben werden. Jedes Elektrodenpaar ist über ein Widerstandsmeßinstrument 31, 32, 33 mit einer Auswerteeinrichtung 34 verbunden, das die im Gewebe des Organismusteils 10 bestehenden Widerstandswerte mißt und der Auswerteeinrichtmag 34 zur Speicherung, Erzeugung der Widerstandsbilder und ihrer Darstellung und Weiterverarbeitung zuleitet. Zu dieser Weiterverarbeitung gehört die therapeutische Behandlung eines bei der Widerstandsmessung festgestellten Karzinoms 21. Im übrigen gilt das zu Fig. 1 Gesagte.

In Fig. 3 ist ein Katheter 35 mit zwei in Schutzhüllen 36, 37 befindlichen Nadeln 38, 39 dargestellt, die in das Katheter 35 ein- und ausfahrbar und über die Radiofrequenzen an ein Karzinom 40 anlegbar sind. Mindestens eine ebenfalls in das Katheter 35 ein- und ausfahrbare Kanüle 41 dient von Hand oder robotergsteuert der Injektion einer geeigneten Flüssigkeit, bspw. einer physiologischen Kochsalzlösung, vor der Applizierung von Radiofrequenzen zur Erhitzung des zwischen den Nadelspitzen befindlichen Organismusgewebes. Dadurch wird der Erhitzungseffekt verstärkt und homogenisiert und damit die Behandlung eines Patienten verkürzt und/oder auf größere Gewebebereiche ausgedehnt. Im übrigen gilt das zu Fig. 1 Gesagte sinngemäß.

Alle in der Beschreibung, den nachfolgenden Ansprüchen und der Zeichnung dargestellten Merkmale können sowohl einzeln als auch in beliebiger Kombination miteinander erfindungswesentlich sein.

### Bezugzeichenliste

- 10: Brust
- 11, 42: Gleichstromquellen
- 12,13: Elektroden
- 14, 15: Doppelpfeile
- 16: Gleichstromkreis
- 17: Meßinstument
- 18: Schalter
- 19: Träger
- 20,34: Auswerteeinrichtung
- 21: Tumor
- 22, 23, 36, 37: Schutzhüllen
- 24, 25, 38, 39: Nadeln
- 26: Steuerung
- 27: Hülle
- 28, 29, 30: Elektrodenpaare
- 31,32,33: Widerstandsmeßinstrumente
- 35: Katheter
- 40: Karzinom
- 41: Kanüle
- X-X: Achse.
- E, E', E": (Querschnitts-) Ebenen

## Patentansprüche

1. Anordnung zum Nachweis, zur differentialdiagnostischen Charakterisierung und zur Therapie von Tumoren, an einem lebenden Organismus oder Organismusteil mit Hilfe von elektrischen Widerstandsbildern, enthaltend:
Mittel zur Zuführung einer Elektrolytlösung in den Organismus/Organismusteil;
mindestens ein Elektrodenpaar, dessen Elektroden im wesentlichen diametral zum Organismus/Organismusteil in einer Querschnittsebene angeordnet werden können, mit dem zumindest nach Zuführung der Elektrolytlösung in unterschiedlicher Orientierung bezüglich des Organismus/Organismusteils Widerstandsignale abgegriffen werden können;
ein Meßgerät zur Umformung der Widerstandssignale in Widerstandswerte;
eine Auswerteeinrichtung, der die Widerstandswerte zur Erzeugung und Abbildung einer zweidimensionalen Widerstandsverteilung des Gewebes des Organismus/Organismusteils in der Querschnittsebene zugeleitet werden können;
Mittel zur Relativbewegung zwischen den Elektroden und dem Organismus/Organismusteil rechtwinklig zur Querschnittsebene und Mittel, die durch Hyperthermieeinwirkung oder Alteration von Membranpotentialen auf das hinsichtlich seiner Widerstandsverteilung abgebildete Gewebe des Organismus/Organismusteils, dessen Widerstandsverteilung abgebildet wurde, einwirken können.

2. Anordnung gemäß Anspruch 1, **dadurch gekennzeichnet, dass** das eine Elektrodenpaar um eine Achse (X-X) schwenkbar angeordnet ist, die rechtwinklig zur Querschnittsebene gerichtet ist.

3. Anordnung gemäß Anspruch 1 oder 2, **dadurch gekennzeichnet, daß** die Mittel zur Zuführung der Elektrolytlösung Kanülen und die Mittel, die durch Hyperthermieeinwirkung oder Alteration von Membranpotentialen auf das Gewebe einwirken, Nadeln sind, die ein- und ausfahrbar an einem Katheter angeordnet sind.

4. Anordnung gemäß Anspruch 3, **dadurch gekennzeichnet, daß** eine Robotersteuerung für die Nadeln und/oder die Kanülen vorgesehen ist.

## Claims

1. Arrangement for detecting, for **characterizing by** differential diagnosis, and for treating tumors of a living organism or a part of organism with the aid of electrical resistance images comprising
means for adding an electrolyte solution to the organism/organism part;
at least one pair of electrodes, the electrodes of which are arranged substantially diametrally to the organism/organism part in a cross-sectional plane, said at least one pair of electrodes being for deriving resistance signals from different orientations relative to the organism/organism part;
a measuring instrument for converting said resistance signals into resistance values;
an evaluation unit into which said resistance values can be fed for generating and imaging a two-dimensional resistance distribution of the tissue of the organism/organism part in the cross-sectional plane; means for relative movement between the electrodes and the organism/organism part at right angles to said cross-sectional plane; and
means which can have an effect on the tissue of the organism/organism part being imaged with respect to its resistance distribution, whereby said resistance distribution was imaged of this tissue, whereby said effect is caused by means of hyperthermia effects or the change of the membrane potentials.

2. An arrangement as claimed in claim 1, **characterized in that** a pair of electrodes is arranged for rotation about an axis (X-X) which is at right angles to the cross sectional plane.

3. An arrangement as claimed in claim 1 or claim 2, **characterized in that** said means for supplying an electrolyte solution are cannulas and said means which have an influence on the tissue by hyperthermia effects or by the change of the membrane potentials are needles which are arranged at a catheter and are extendible into and retractable from out said catheter.

4. Arrangement as claimed in claim 3, **characterized in that** a robot control is provided for said needles and/or said cannulas.

## Revendications

1. Configuration de l'agencement pour la détection, la caractérisation par diagnostic différentiel et le traitement de tumeurs sur tout ou partie d'un organisme vivant à l'aide de l'imageries de la résistance électrique, comprenant:
des moyens assurant l'entrée d'une solution électrolytique dans tout ou partie d'un organisme;
au moins une paire d'électrodes, lesquelles, à hauteur de la coupe transversale, peuvent être disposées pour l'essentiel dans une position diamétralement opposée à l'organisme ou à la partie concernée d'un organisme pour permettre, après avoir ajouté une solution électrolytique, de capter et de reconnaître dans diverses orientations les signaux de résistance;
un dispositif de mesure pour transformer les signaux de résistance captés en valeurs de résistance;
un système d'interprétation auquel on peut transmettre les valeurs de résistance détectées pour obtenir et représenter sous forme d'images une répartition bidimensionnelle de la résistance du tissu dans un organisme/une partie d'un organisme à hauteur de la coupe transversale;
des moyens assurant le mouvement relatif entre électrodes et organisme/partie d'un organisme de manière perpendiculaire par rapport au niveau de la coupe transversale ainsi
que des moyens qui, par leur action hyperthermique ou par altération de potentiels de membrane, peuvent exercer une influence sur le tissu de l'organisme/de la partie concernée de l'organisme dont la répartition des résistances a été représentée sous forme d'image.

2. La configuration suivant la revendication 1 est **caractérisée en ce que** la paire d'électrode est disposée de manière à pivoter autour d'une axe (X-X) perpendiculaire par rapport au plan de la coupe transversale.

3. La configuration suivant la revendication 1 ou 2 est **caractérisée en ce que** les moyens assurant l'amenée de la solution électrolytique sont des canules et que les moyens exerçant un influence sur le tissu par hypothermie ou altération de potentiels de membrane sont des aiguilles disposées sur un cathéter de manière à pouvoir les faire entrer ou sortir.

4. La configuration suivant la revendication 3 est **caractérisée en ce qu'**un système Robot assure la commande des aiguilles et/ou des canules.
